# EUROPÄISCHE PATENTANMELDUNG

(11) **EP 2 441 410 A1**
(43) Veröffentlichungstag der Anmeldung: **18.04.2012**
(21) Anmeldenummer: 11183895.9
(22) Anmeldetag: 05.10.2011
(51) Int. Cl.: A61B 19/00

(54) **Verfahren zum Erstellen eines medizinischen Bildes und medizinischer Arbeitsplatz**

(30) Priorität: 13.10.2010 DE 102010042372
(71) Anmelder: KUKA Laboratories GmbH, 86165 Augsburg (DE)
(72) Erfinder: Jacob, Dirk, 87616 Marktoberdorf (DE); Kuschel, Martin, 80339 München (DE); Neff, Thomas, 80339 München (DE); Ortmaier, Tobias, Prof. Dr.-Ing., 30966 Hemmingen (DE); Ueberle, Marc-Walter, 86316 Friedberg (DE)
(74) Vertreter: Patentanwälte Funk & Böss GbR

(57) **Zusammenfassung**

Die Erfindung betrifft ein Verfahren zum Erstellen eines medizinischen Bildes mittels eines Bildes, das mit einer in das Innere eines Lebewesens (31) eingeführten Kamera (6) vom Inneren des Lebewesens (31) während eines medizinischen Eingriffs aus dem Blickwinkel (8) der Kamera (6) ein Bild aufgenommen wird. Durch Fusionieren des dem Bild zugeordneten Bilddatensatzes mit einem vor dem Eingriff präoperativ aufgenommenen Volumendatensatzes vom Inneren des Lebewesens (31) wird ein Volumendatensatz erzeugt. Der Volumendatensatz wird derart gedreht und/oder verschoben, dass das dem Volumendatensatz zugeordnete virtuelle Bild (21) vom Inneren des Lebewesens (31) aus einem vom Blickwinkel (8) der Kamera (6) unterschiedlichen virtuellen Blickwinkel (28) auf einer Anzeigevorrichtung (38) angezeigt wird und/oder der virtuelle Blickabstand des dem Volumendatensatz zugeordneten virtuellen Bildes (21) vom Inneren des Lebewesens (31) modifiziert wird.

## Beschreibung

Die Erfindung betrifft ein Verfahren zum Erstellen eines medizinischen Bildes und einen medizinischen Arbeitsplatz.

In der robotergestützten bzw. telemanipulierten Chirurgie, wie es u.A. in der US 6,799,065 B1 oder der US 6,522,906 B1 offenbart ist, werden medizinische Instrumente mittels Roboterarmen bewegt, die wiederum ferngesteuert mittels einer Eingabekonsole manuell von einem Arzt bzw. Chirurg bewegt werden. Um einen visuellen Einblick vom Operationssitus, also von dem Gebiet eines Lebewesens, das aktuell mittels der medizinischen Instrumente behandelt wird, zu erhalten, kann in das Lebewesen z.B. mittels eines weiteren, fernsteuerbaren Roboterarms eine an einem Endoskop angeordnete Kamera eingeführt werden, welche Bilder vom aktuellen Operationssitus aufnimmt. Diese Bilder können an einer insbesondere als 3D-Bildschirm ausgebildeten Anzeigevorrichtung der Eingabekonsole dargestellt werden. Mittels des Bildes werden z.B. die Anatomie, aber auch die medizinischen Instrumente dargestellt.

Die US 6,892,090 B2 offenbart ein medizinisches Navigationssystem, welches eine dreidimensionale Darstellung eines Operationssitus aus dem Blickwinkel eines in das Lebewesen eingeführten medizinischen Instruments simuliert. Es werden z.B. mittels Computertomographie-, Ultraschall- oder Magnetresonanzgeräten erzeugte prä- oder intraoperativ erstellter Volumenbilddaten vom Lebewesen verwendet.

Aufgabe der Erfindung ist es, ein Verfahren anzugeben, welches es einem Operateur während eines Eingriffs in ein Lebewesen erlaubt, eine verbesserte Darstellung vom Operationssitus zu erhalten.

Die Aufgabe der Erfindung wird gelöst durch ein Verfahren zum Erstellen eines medizinischen Bildes, aufweisend folgende Verfahrensschritte:
- Erstellen eines Bildes mittels einer in das Innere eines Lebewesens eingeführten Kamera vom Inneren des Lebewesens während eines medizinischen Eingriffs aus dem Blickwinkel der Kamera,
- Erstellen eines Volumendatensatzes durch Fusionieren des dem Bild zugeordneten Bilddatensatzes mit einem vor dem Eingriff präoperativ aufgenommenen Volumendatensatzes vom Inneren des Lebewesens, und
- virtuelles Drehen des Volumendatensatzes derart, dass das dem Volumendatensatz zugeordnete Bild vom Inneren des Lebewesens aus einem vom Blickwinkel der Kamera unterschiedlichen virtuellen Blickwinkel auf einer Anzeigvorrichtung angezeigt wird.

Zusätzlich oder alternativ kann noch ein Verschieben des Volumendatensatzes derart vorgesehen sein, dass der virtuelle Blickabstand des dem Volumendatensatz zugeordneten virtuellen Bildes vom Inneren des Lebewesens modifiziert wird.

Gemäß dem erfindungsgemäßen Verfahren wird demnach u.A. ein Volumendatensatz vom Inneren eines Lebewesens erstellt und zwar durch Fusionieren bzw. Überlagern zweier Bilder bzw. deren zugeordneter Datensätzen. Einer der Datensätze, der präoperativ aufgenommene Volumenbilddatensatz wurde vor dem Eingriff aufgenommen insbesondere mit einem bildgebenden medizinischen Gerät. Geeignete bildgebende medizinische Geräte sind z.B. Computertomographie-, Magnetresonanz- oder gegebenenfalls auch Ultraschallgeräte, die eingerichtet sind, ein dreidimensionales Bild bzw. einen Volumenbilddatensatz zu erzeugen. Der Bilddatensatz wird mittels der Kamera während des Eingriffs aufgenommen. Die Kamera ist in das Innere des Lebewesens eingeführt und kann gegebenenfalls ein zwei- oder auch ein dreidimensionales Bild erzeugen. Somit umfasst der erzeugte Volumendatensatz eine Information über den aktuellen inneren Zustand des Operationssitus.

Der erzeugte Volumendatensatz wird anschließend gedreht und das diesem Volumendatensatz zugeordnete Bild wird auf einer Anzeigevorrichtung dargestellt. Aufgrund der Drehung des Volumendatensatzes erscheint das dargestellte Bild aus einem anderen Blickwinkel als das mit der Kamera aufgenommene Bild. Somit kann z.B. eine den medizinischen Eingriff durchführende Person sich einen besseren Überblick über den aktuellen Operationssitus verschaffen.

Das Erstellen des Volumendatensatzes kann mittels Bildregistrierung des Bilddatensatzes und des präoperativ aufgenommnen Volumenbilddatensatzes erfolgen. Bildregistrierung als solche ist dem Fachmann im Prinzip bekannt und stellt ein Verfahren in der digitalen bzw. diskreten Bildverarbeitung dar, das verwendet wird, zwei oder mehrere Bilder derselben Szene, oder zumindest ähnlicher Szenen bestmöglich oder zumindest ausreichend gut in Übereinstimmung miteinander zu bringen. Dabei kann eines der Bilder bzw. dessen Bilddatensatz als Referenzbild festgelegt werden. Das andere Bild bzw. die anderen Bilder sind dann sogenannte Objektbilder. Anschließend kann eine Transformation berechnet werden, die die Objektbilder bestmöglich, zumindest jedoch ausreichend gut, an das Referenzbild anpasst. Als Referenzbild kann insbesondere das mittels der Kamera aufgenommene Bild verwendet werden.

Um die Qualität des erzeugten Volumenbilddatensatzes zu verbessern, kann nach einer Variante des erfindungsgemäßen Verfahrens eine Bewegung des Lebewesens ermittelt und diese Bewegung beim Erstellen des Volumenbilddatensatzes berücksichtigt werden. Um die Bewegung des Lebewesens zumindest teilweise zu kompensieren, kann z.B. ein dynamisches Modell beim Erstellen des Volumendatensatzes verwendet werden. Dies kann z.B. dadurch erreicht werden, dass 4D-Bilddaten bzw. zeitlich nacheinander folgende, aufgezeichnete, mittels der Kamera erstellter Bilddatensätze verwendet werden. Dies ermöglicht die Darstellung insbesondere sich periodisch bewegender Organe, wie z.B. des Herzens des Lebewesens. Beispielsweise die Bildregistrierung der Bewegung des Herzens mit der aktuellen Szene kann auch durch Einsatz eines EKG-Triggers erleichtert werden.

Die Kamera wird z.B. mittels eines Endoskops in das Innere des Lebewesens eingeführt. Die Kamera ist dann am Endoskop angeordnet, insbesondere an einem Ende, das in das Lebewesen eingeführt ist, befestigt oder auch integriert.

Der medizinische Eingriff erfolgt insbesondere mittels wenigstens eines in das Lebewesen eingeführten medizinischen Instruments. Bevorzugt werden zwei medizinische Instrumente für den Eingriff und ein mit der Kamera versehenes Endoskop verwendet.

Der erzeugte Volumendatensatz kann manuell gedreht werden, um den virtuellen Blickwinkel einzustellen.

Nach einer bevorzugten Ausführungsform des erfindungsgemäßen Verfahrens weist dieses ein automatisches Drehen des virtuellen Volumendatensatzes auf, um den virtuellen Blickwinkel aufgrund eines vorab festgelegten oder festlegbaren Kriteriums automatisch einzustellen. Vorzugsweise wird der erzeugte Volumendatensatzes derart automatisch gedreht, sodass der virtuelle Blickwinkel in Richtung des wenigstens einen medizinischen Instruments gerichtet ist. Für ein intuitives Betrachten des Operationssitus, also des Bereichs, der gegenwärtig mittels des wenigstens einen medizinischen Instruments behandelt wird, ist es wünschenswert, dass dieser vom Blickwinkel der medizinischen Instrumente betrachtet werden kann. Aus Platzgründen ist es jedoch in der Regel nicht möglich, die Kamera derart in das Lebewesen einzuführen, sodass das mit der Kamera aufgenommene Bild von diesem Blickwinkel erstellt wird. Aufgrund dieser Variante des erfindungsgemäßen Verfahrens ist es möglich, das Bild von einem beliebigen Blickwinkel aufzunehmen. Aufgrund der Drehung ergibt sich der virtuelle Blickwinkel in Richtung des wenigstens einen medizinischen Instruments.

Die medizinischen Instrumente und/oder das Endoskop bzw. die Kamera können direkt manuell in das Lebewesen eingeführt werden.

Nach einer bevorzugten Ausführungsform des erfindungsgemäßen Verfahrens weist dies ein Einführen des wenigstens einen medizinischen Instruments und der Kamera in das Innere des Lebewesens mittels Roboterarme auf, welche manuell mittels einer Eingabevorrichtung bewegt werden, sodass die Roboterarme einer der manuellen Bewegung der Eingabevorrichtung entsprechende Bewegung durchführen.

Ein weiterer Aspekt der Erfindung betrifft daher einen medizinischen Arbeitsplatz zum Behandeln eines Lebewesens mittels wenigstens eines medizinischen Instruments, aufweisend
- mehrere Roboterarme, die jeweils mehrere mittels Gelenken verbundene Glieder, Antriebe zum Bewegen der Glieder, und eine Befestigungsvorrichtung aufweisen, wobei wenigstens eine der Befestigungsvorrichtungen vorgesehen ist, mit dem medizinischen Instrument zum Behandeln des Lebewesens versehen zu werden, und eine der Befestigungsvorrichtungen vorgesehen ist, mit einem Endoskop, an dem eine Kamera angeordnet ist, versehen zu werden,
- wenigstens eine mit den Antrieben gekoppelte Steuervorrichtung, welche eingerichtet ist, Signale zum Ansteuern der Antriebe zu erzeugen, damit die Befestigungsvorrichtungen den Signalen zugeordnete Bewegungen durchführen, und
- eine mit der Steuervorrichtung gekoppelte Bedienkonsole, die eine mit der Steuervorrichtung gekoppelte manuelle Eingabevorrichtung und eine mit der Steuervorrichtung gekoppelte Anzeigevorrichtung aufweist, wobei die Steuervorrichtung eingerichtet ist, aufgrund eines Bildes vom Inneren des Lebewesens, das mit der während eines medizinischen Eingriffs in das Innere des Lebewesens eingeführten Kamera aufgenommen wurde, einen Volumendatensatz durch Fusionieren des dem Bild zugeordneten Bilddatensatzes mit einem vor dem Eingriff präoperativ aufgenommenen Volumendatensatzes vom Inneren des Lebewesens zu erstellen, und den Volumendatensatz derart zu drehen, um das dem Volumendatensatz zugeordnete virtuelle Bild vom Inneren des Lebewesens aus einem vom Blickwinkel der Kamera unterschiedlichen virtuellen Blickwinkel auf der Anzeigvorrichtung anzuzeigen.

Der erfindungsgemäße medizinische Arbeitsplatz umfasst demnach die Roboterarme, die mittels der manuellen Eingabevorrichtung der Bedienkonsole telemanipulierbar sind, um somit das Lebewesen mittels des wenigstens einen medizinischen Instruments zu behandeln. Aufgrund des angezeigten virtuellen Bildes kann die das Lebewesen behandelnde Person gegebenenfalls den Operationssitus aus einem besser geeigneten Blickwinkel betrachten.

Die Steuervorrichtung kann vorzugsweise eingerichtet sein, den Volumendatensatz derart automatisch zu drehen, dass der virtuell Blickwinkel in Richtung des wenigstens einen medizinischen Instruments gerichtet ist. Somit wird es ermöglicht, eine verbesserte Hand-Auge-Koordination des wenigstens eines medizinischen Instruments herzustellen.

Es ist auch möglich, das virtuelle Bild an die Bewegungen des wenigstens einen medizinischen Instruments zu koppeln. Hat man beispielsweise ein virtuelles Modell der Organe und gegebenenfalls der Instrumente, das mittels der Kameradaten und gegebenenfalls weiteren Sensordaten (z.B. der Positionsdaten der Instrumente) aktualisiert werden kann, ist es möglich, eine "virtuelle Kamera", d.h. den virtuellen Blickwinkel beliebig zu positionieren, d.h. man löst sich von den Freiheitsgrad-Beschränkungen der Mensch-Maschine-Schittstelle.

Für eine verbesserte Hand-Auge-Koordination insbesondere bei Teleoperationssystemen durch virtuelle Ansichten des Operationsgebietes können je nach Ausführungsform des erfindungsgemäßen folgende Verfahrensschritte durchgeführt werden:
Aufzeichnung präoperativer Bilder (CT, MRT, US), d.h. des präoperativ aufgenommenen Volumenbilddatensatz, eventuell Durchführung einer OP-Planung mit Segmentierung interessierender Strukturen.
Aufzeichnung wenigstens eines intraoperativ aufgenommenen Bildes insbesondere mit Hilfe eines Endoskops.

Gegebenfalls Registrierung der prä- und interoperativen Bilddaten zur Erstellung einer virtuellen Szene des OP-Gebiets, d.h. des erzeugten Volumenbilddatensatzes. Hierbei können insbesondere Verfahren der elastischen Registrierung eingesetzt werden, um z.B. eine Verschiebung innerhalb der Anatomie des Lebewesens auszugleichen.

Gegebenfalls Hervorhebung der interessierenden Strukturen aus der eventuell durchgeführten OP-Planung.

Gegebenfalls Kopplung des mit der Kamera aufgenommenen Bildes an die Bewegungen der Instrumente für ein verbesserte Hand-Auge-Koordination.

Gegebenfalls Erweiterung des Bildes der Kamera um die virtuelle Szene, wenn z.B. Gefahr besteht, dass die Hand-Auge-Koordination eingeschränkt werden kann. Dies ist insbesondere bei Verdeckung der medizinischen Instrumente der Fall (Verdeckung der Instrumente kann erkannt werden; Erkennung insbesondere der Instrumentenspitzen im Endoskopbild) oder wenn die Instrumente so bewegt werden, dass die Kamera diese Bewegungen nicht ausgleichen kann (Erkennung, dass die Relation Instrument-Kamera nicht eingehalten werden kann) und die Hand-Augen-Koordination verloren geht.

Es ist möglich, dass das Bild der Kamera komplett von der virtuellen Szene ersetzt wird. Es entsteht eine virtuelle Replatzierung der Kamera. In diesem Fall kann auch das Bild der Kamera zusätzlich eingeblendet werden, um durch eventuelle Fehler des virtuellen Bildes das Lebewesen nicht zu gefährden.

Die Zuverlässigkeit des Modells/ der gerenderten Darstellung kann farblich kodiert werden (Bereiche, die direkt von der Kamera erfasst werden, sollten zuverlässiger zu modellieren zu sein, als verdeckte Bereiche). Eine Möglichkeit, die Zuverlässigkeit von gerenderten Strukturen zu modellieren, ist die Farbsättigung unzuverlässiger Bereiche zu vermindern, sodass diese "graustichiger" werden. Es ist auch möglich, die Bildschärfe in Abhängigkeit der Unzuverlässigkeit zu reduzieren.

Es können auch anatomische Atlanten zur Registrierung mit intraoperativen Bilddaten und Erweiterung des Operationsbereiches eingesetzt werden. Ein dynamisches Modell zur Generierung der virtuellen Szene des OP-Gebietes kann auch eingesetzt werden. Eine Quelle können 4D-Bilddaten bzw. aufgezeichnete Daten der Kamera sein. Dies ermöglicht die Darstellung insbesondere sich periodisch bewegender Organe. Ein Beispiel hierfür ist das Herz. Die Registrierung der Bewegung des Herzens mit der aktuellen Szene kann durch Einsatz eines EKG-Triggers erleichtert werden (Aufzeichnung des EKG bei präoperativen 4D-Bilddaten, Registrierung der des Triggers mit aktuellen EKG-Daten)

Alternative zur Benutzung der virtuellen Szene (anstatt zur Beibehaltung der Hand-Augen-Koordination) kann bei Verdeckung mit Hilfe der virtuellen Szene die "Kameraposition", d.h. der virtuell Blickwinkel beliebig variiert werden, um Zusatzinformationen zu erhalten.

Ein Ausführungsbeispiel der Erfindung ist exemplarisch in den beigefügten schematischen Figuren dargestellt. Es zeigen:
Fig. 1 einen Operationssitus,
Fig. 2 ein virtuelles Bild vom Operationssitus und
Fig. 3 einen medizinischen Arbeitsplatz.

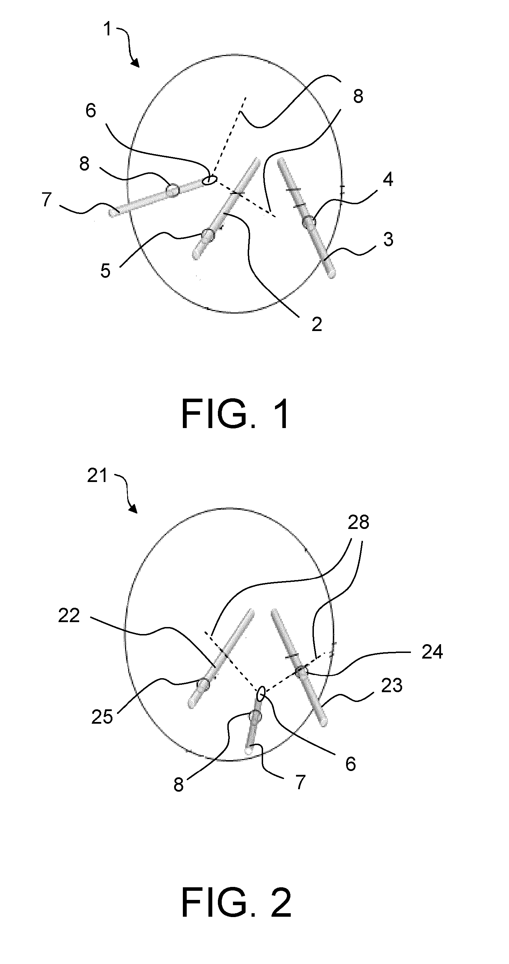

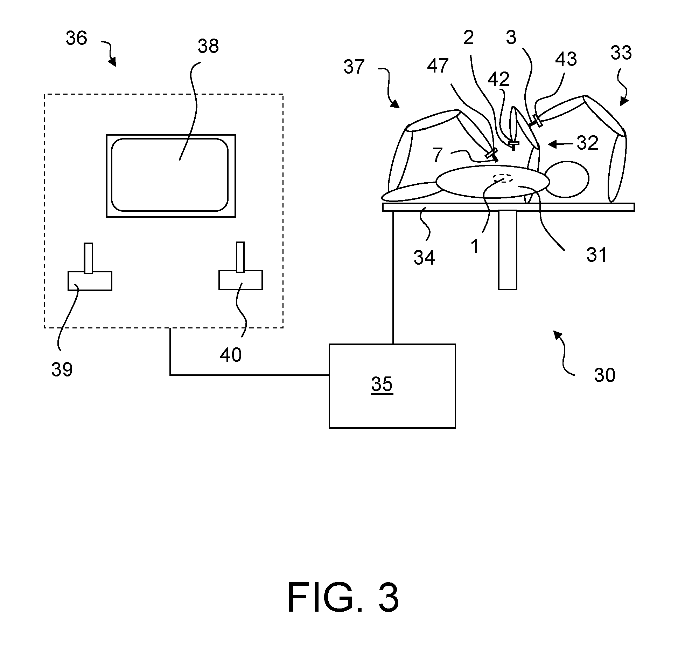

Die Fig. 1 zeigt einen Operationssitus 1, also die Umgebung eines medizinischen Eingriffs in ein in der Fig. 3 gezeigtes Lebewesen 31, das z.B. auf einer Patientenliege 34 liegt.

Das Lebewesen 31 wird im Falle des vorliegenden Ausführungsbeispiels einem medizinischen Eingriff, insbesondere einem minimal-invasiven medizinischen bzw. chirurgischen Eingriff unterzogen. Dazu verwendet ein in den Figuren nicht näher dargestellter Arzt, der den Eingriff durchführt, zwei medizinische Instrumente 2, 3, die der Arzt in das Innere des Lebewesens 31 einführt.

Im Falle des vorliegenden Ausführungsbeispiels wurden dem Fachmann im Prinzip bekannte Trokare verwendet, um Zugänge für die medizinischen Instrumente 2, 3 in das Innere des Lebewesens 31 zu schaffen, die durch Tubusse 4, 5 offen gehalten werden. Durch die Tubusse 4, 5 hat im Falle des vorliegenden Ausführungsbeispiels der Arzt die beiden medizinischen Instrumente 2, 3 in das Innere des Lebewesens 31 geführt, um den Eingriff durchzuführen.

Um den Eingriff besser beobachten zu können, verwendet der Arzt eine Kamera 6, die an einem Endoskop 7 angeordnet ist, insbesondere an dessen Spitze befestigt oder integriert ist. Der Zugang für das Endoskop 7 wurde ebenfalls mittels eines Trokars geschaffen und wird mittels eines weiteren Tubus 8, durch den der Arzt das Endoskop 7 in das Innere des Lebewesens 31 führt, offen gehalten. Mittels der in das Innere des Lebewesens 31 geführten Kamera 6 wird es somit dem Arzt ermöglicht, seinen mit den medizinischen Instrumenten 2, 3 durchgeführten Eingriff zu beobachten. Das Sichtfeld der Kamera 6 bzw. der Blickwinkel 8 der Kamera 6 ist in der Fig. 1 mittels zweier gestrichelter Linien angedeutet. Außerdem ist das Endoskop 7, an dem die Kamera 6 befestigt ist, seitlich relativ zu den beiden medizinischen Instrumenten 2, 3 in das Innere des Lebewesen 31 eingeführt.

Im Falle des vorliegenden Ausführungsbeispiels werden die medizinischen Instrumente 2, 3 und das Endoskop 7 mittels in der Fig. 3 gezeigten Roboterarme 32, 33, 37 eines medizinischen Arbeitsplatzes 30 bewegt. Die Roboterarme 32, 33, 37 sind mit einer Steuervorrichtung 35 des medizinischen Arbeitsplatzes 30 gekoppelt, die wiederum mit einer Bedienkonsole 36 des medizinischen Arbeitsplatzes 30 verbunden ist. Die Bedienkonsole 36 umfasst ein Anzeigevorrichtung 38, die insbesondere eingerichtet ist, dreidimensionale Bilder anzuzeigen, und manuelle Eingabevorrichtungen 39, 40, mittels derer der Arzt die Roboterarme 32, 33, 37 telemanipulieren kann, wie dies im Prinzip dem Fachmann bekannt ist.

Im Falle des vorliegenden Ausführungsbeispiels umfasst jeder der Roboterarme 32, 33, 37 mehrere Glieder, die über Gelenke verbunden sind, und eine Befestigungsvorrichtung 42, 43, 47. An den Befestigungsvorrichtungen 42, 43 der Roboterarme 32, 33 sind die beiden medizinischen Instrumente 2, 3 und an der Befestigungsvorrichtung 47 des Roboterarms 37 ist das Endoskop 7 befestigt.

Die Antriebe der Roboterarme 32, 33, 37, die insbesondere elektrische Antriebe sind, sind mit der Steuervorrichtung 35 verbunden. Die Steuervorrichtung 35, die z.B. als ein Rechner ausgeführt ist, ist eingerichtet, insbesondere mittels eines Rechenprogramms die Antriebe derart anzusteuern, sodass die Roboterarme 32, 33, 37 ihre Befestigungsvorrichtungen 42, 43, 47 und somit die medizinischen Instrumente 2, 3 und das Endoskop 7 gemäß einer mittels der manuellen Eingabevorrichtungen 39, 40 bewegen. Gegebenfalls ist die Steuervorrichtung 35 auch derart eingerichtet, dass sie die Bewegungen der Roboterarme 32, 33, 37 bzw. der Antriebe regelt, sodass im vorliegenden Fall der Begriff "Steuern" auch "Regeln" umfassen soll.

Auch die medizinischen Instrumente 2, 3 und die Kamera 6 sind mit der Steuervorrichtung 35 verbunden, sodass der Arzt die medizinischen Instrumente 2, 3 mittels der Bedienkonsole 36 aktivieren kann bzw. das mittels der Kamera 6 aufgenommene Bild mittels der Anzeigevorrichtung 38 betrachten kann.

Im Falle des vorliegenden Ausführungsbeispiels wurde vor dem Eingriff ein dreidimensionales Bild vom Inneren des Lebewesens 31 erstellt. Das dreidimensionale Bild wurde z.B. mittels eines dem Fachmann im Allgemeinen bekannten bildgebenden medizinischen Gerätes, beispielsweise mittels eines Computertomographie-, Ultraschall- oder Magnetresonanzgerätes erstellt. Der dazugehörigen Volumenbilddatensatz ist beispielsweise in der Steuervorrichtung 35 gespeichert.

Wie bereits erwähnt, kann der Arzt im Falle des vorliegenden Ausführungsbeispiels mittels der in das Lebewesen 31 eingeführten Kamera 6 während des Eingriffs, also intraoperativ, Bilder vom Operationssitus 1 erstellen. Auf der Steuervorrichtung 35 läuft außerdem ein Rechenprogramm, das den intraoperativ aufgenommenen Bilddatensatz, der dem mittels der Kamera 6 erstellten Bildes zugeordnet ist, mit dem präoperativ aufgenommenen Volumenbilddatensatz verknüpft, überlagert bzw. fusioniert, sodass ein weitere Volumenbilddatensatz entsteht, der sowohl eine Information vom präoperativ aufgenommenen Volumenbilddatensatz als auch vom intraoperativ aufgenommenen Bilddatensatz umfasst. Um die Überlagerung bzw. Fusionierung zu ermöglichen, wird beispielsweise eine dem Fachmann im Prinzip bekannte Bildregistrierung verwendet, deren zugeordnete Rechenvorschrift insbesondere auf der Steuervorrichtung 35 läuft.

Bildregistrierung im Allgemeinen ist ein Verfahren in der digitalen bzw. diskreten Bildverarbeitung, die verwendet wird, zwei oder mehrere Bilder derselben Szene, oder zumindest ähnlicher Szenen, im vorliegenden Ausführungsbeispiels also vom Operationssitus 1, bestmöglich, zumindest jedoch ausreichend gut in Übereinstimmung miteinander zu bringen. Dabei kann eines der Bilder bzw. dessen Bilddatensatz als Referenzbild festgelegt werden. Das andere Bild bzw. die anderen Bilder sind dann sogenannte Objektbilder. Anschließend kann eine Transformation berechnet werden, die die Objektbilder bestmöglich, zumindest jedoch ausreichend gut, an das Referenzbild anpasst. Als Referenzbild kann insbesondere das mittels der Kamera 6 aufgenommene Bild verwendet werden.

Die zu registrierenden Bilder unterscheiden sich in der Regel voneinander, weil sie von unterschiedlichen Positionen, zu unterschiedlichen Zeitpunkten oder mit unterschiedlichen Sensoren aufgenommen wurden. Im Fall des vorliegenden Ausführungsbeispiels wird insbesondere die elastische Registrierung verwendet.

Es ist auch möglich, für die Bildregistrierung anatomische Atlanten zu verwenden, die z.B. in der Steuervorrichtung 35 gespeichert sind.

Um eine Bewegung des Lebewesens 31 zumindest teilweise zu kompensieren, kann es auch vorgesehen sein, dass ein dynamisches Modell beim Erstellen des weiteren Volumendatensatzes verwendet wird. Dies kann z.B. dadurch erreicht werden, dass 4D-Bilddaten bzw. zeitlich nacheinander folgende, aufgezeichnete, mittels der Kamera 6 erstellte Bilddatensätze verwendet werden. Dies ermöglicht die Darstellung insbesondere sich periodisch bewegender Organe, wie z.B. des Herzens des Lebewesens 31. Die Bildregistrierung der Bewegung des Herzens mit der aktuellen Szene kann auch durch Einsatz eines EKG-Triggers erleichtert werden (Aufzeichnung eines nicht näher dargestellten EKGs bei präoperativen 4D-Bilddaten, Bildregistrierung der des Triggers mit aktuellen EKG-Daten).

Im Falle des vorliegenden Ausführungsbeispiels ist es anschließend vorgesehen, automatisch den weiteren Volumenbilddatensatz, also den Volumendatensatz, der sowohl eine Information über das präoperativ als auch des intraoperativ aufgenommene Bildes umfasst, derart zu drehen, dass es einem virtuellen Blickwinkel 28 in Richtung der beiden medizinischen Instrumente 2, 3 entspricht. Aufgrund der Stellungen der Roboterarme 32, 33, 37 bzw. der daraus resultierenden Lagen der medizinischen Instrumente 2, 3 und des Endoskops 7 ist es der Steuervorrichtung 35 möglich, den virtuellen Blickwinkel 28 zu ermitteln.

Das Bild 21 mit dem virtuellen Blickwinkelfeld 28, das in der Fig. 2 veranschaulicht ist, wird dem Arzt auf der Anzeigevorrichtung 38 des Bedienkonsole 36 angezeigt. Der virtuelle Blickwinkel 28 ist in der Fig. 2 mittels gestrichelter Linien angedeutet. Die Abbilder der medizinischen Instrumente 2, 3 sind mit den Bezugszeichen 22, 23 und die Abbilder der Tubusse 4, 5 sind mit den Bezugszeichen 24, 25 versehen. In der Fig. 2 ist außerdem das Endoskop 7, dessen zugeordneter Tubus 8 und die Kamera 6 derart gezeigt, als ob das Endoskop 7 zwischen den beiden medizinischen Instrumenten 2, 3 in das Lebewesen 31 eingeführt wäre, um den virtuellen Blickwinkel 28 zu erlauben.

Im Falle des vorliegenden Ausführungsbeispiels kann es noch vorgesehen sein, kontinuierlich den weiteren Volumendatensatz zu berechnen und somit den virtuellen Blickwinkel 28 einer Bewegung der medizinischen Instrumente 2, 3 bzw. des Endoskops 7 anzupassen.

Im Falle des vorliegenden Ausführungsbeispiels kann es noch vorgesehen sein, dass bestimmte Bereiche des Bildes 21 beispielsweise farblich kodiert bzw. hervorgehoben sind. Diese Bereiche können z.B. Bereiche sein, die direkt von der Kamera 6 erfasst werden. Diese Bereiche können gegebenenfalls zuverlässiger modelliert werden als z.B. von den medizinischen Instrumenten 2, 3 verdeckte Bereiche. Eine Möglichkeit, die Zuverlässigkeit von gerenderten Strukturen zu modellieren, ist die Farbsättigung unzuverlässiger Bereiche zu vermindern, sodass diese "graustichiger" werden. Es ist auch möglich, die Bildschärfe in Abhängigkeit der Unzuverlässigkeit zu reduzieren.

Im Falle des vorliegenden Ausführungsbeispiels ist die Steuervorrichtung 35 derart ausgeführt, dass es den weiteren Volumenbilddatensatz derart dreht, dass der resultierende bzw. virtuelle Blickwinkel 28 automatisch zwischen den beiden medizinischen Instrumenten 2, 3 gelegt wird.

Es ist aber auch möglich, dass der virtuelle Blickwinkel 28 beliebig z.B. vom Arzt gedreht werden kann. Es ist auch möglich, dass der Arzt zwischen dem Bild 21 und dem mittels der Kamera 6 erstellten Bild umschalten kann, sodass das von der Kamera 6 erstellte Bild zusammen mit dem virtuellen Blick auf der Anzeigevorrichtung 38 dargestellt wird.

Im beschriebenen Ausführungsbeispiel werden die medizinischen Instrumente 2, 3 und das Endoskop 7 mittels der Roboterarme 2 3, 7 bewegt. Dies ist nicht absolut nötig, es ist auch vorstellbar, dass der Arzt die medizinischen Instrumente 2, 3 und/oder das Endoskop 7 direkt manuell bedient.

Die Berechnung des weiteren Volumenbilddatensatzes, der eine Information über den präoperativ aufgenommenen Volumenbilddatensatz und das intraoperativ aufgenommene Bild umfasst, braucht nicht notwendigerweise mittels der Steuervorrichtung 35 errechnet zu werden. Es kann auch eine separate Rechenvorrichtung verwendet werden.

Zusätzlich oder alternativ zum Drehen des virtuellen Blickwinkels 28 kann auch ein Verschieben des weiteren Volumendatensatzes derart vorgesehen sein, dass der virtuelle Blickabstand des dem weiteren Volumendatensatz zugeordneten virtuellen Bildes 21 vom Inneren des Lebewesens 31 modifiziert wird, wodurch ein "virtuelles Zoomen" realisiert wird.

## Patentansprüche

1. Verfahren zum Erstellen eines medizinischen Bildes, aufweisend folgende Verfahrensschritte:
- Erstellen eines Bildes mittels einer in das Innere eines Lebewesens (31) eingeführten Kamera (6) vom Inneren des Lebewesens (31) während eines medizinischen Eingriffs aus dem Blickwinkel (8) der Kamera (6),
- Erstellen eines Volumendatensatzes durch Fusionieren des dem Bild zugeordneten Bilddatensatzes mit einem vor dem Eingriff präoperativ aufgenommenen Volumendatensatzes vom Inneren des Lebewesens (31), und
- Drehen und/oder Verschieben des Volumendatensatzes derart, dass das dem Volumendatensatz zugeordnete virtuelle Bild (21) vom Inneren des Lebewesens (31) aus einem vom Blickwinkel (8) der Kamera (6) unterschiedlichen virtuellen Blickwinkel (28) auf einer Anzeigvorrichtung (38) angezeigt wird und/oder der virtuelle Blickabstand des dem Volumendatensatz zugeordneten virtuellen Bildes (21) vom Inneren des Lebewesens (31) modifiziert wird.

2. Verfahren nach Anspruch 1, aufweisend Erstellen des Volumenbilddatensatzes aufgrund einer Bildregistrierung des Bilddatensatzes und des präoperativ aufgenommnen Volumenbilddatensatzes und/oder aufweisend Ermitteln einer Bewegung des Lebewesens (31) und Berücksichtigen der Bewegung des Lebewesens (31) beim Erstellen des Volumenbilddatensatzes.

3. Verfahren nach Anspruch 1 oder 2, aufweisend manuelles Drehen des virtuellen Volumendatensatzes, um den virtuellen Blickwinkel (28) einzustellen.

4. Verfahren nach Anspruch 1 oder 2, aufweisend automatisches Drehen des virtuellen Volumendatensatzes, um den virtuellen Blickwinkel (28) aufgrund eines vorab festgelegten oder festlegbaren Kriteriums automatisch einzustellen.

5. Verfahren nach einem der Ansprüche 1 bis 4, aufweisend Einführen der Kamera (6) in das Lebewesen (31) mittels eines Endoskops (7).

6. Verfahren nach einem der Ansprüche 1 bis 5, aufweisend Einführen wenigstens eines medizinischen Instruments (2, 3) in das Innere des Lebewesens (31).

7. Verfahren nach Anspruch 6, aufweisend automatisches Drehen des virtuellen Volumendatensatzes derart, sodass der virtuelle Blickwinkel (28) in Richtung des wenigstens einen medizinischen Instruments (2, 3) gerichtet ist.

8. Verfahren nach Anspruch 7, aufweisend Einführen des wenigstens einen medizinischen Instruments (2, 3) und der Kamera (6) in das Innere des Lebewesens (31) mittels Roboterarme (32, 33, 37), welche manuell mittels einer Eingabevorrichtung (39, 40) bewegt werden, sodass die Roboterarme (32, 33, 37) einer der manuellen Bewegung der Eingabevorrichtung (32, 33, 37) entsprechende Bewegung durchführen.

9. Medizinischer Arbeitsplatz zum Behandeln eines Lebewesens (31) mittels wenigstens eines medizinischen Instruments (2, 3), aufweisend
- mehrere Roboterarme (2, 3, 7), die jeweils mehrere mittels Gelenken verbundene Glieder, Antriebe zum Bewegen der Glieder, und eine Befestigungsvorrichtung (42, 43, 47) aufweisen, wobei wenigstens eine der Befestigungsvorrichtungen (42, 43) vorgesehen ist, mit dem medizinischen Instrument (2, 3) zum Behandeln des Lebewesens (31) versehen zu werden, und eine der Befestigungsvorrichtungen (47) vorgesehen ist, mit einem Endoskop (7), an dem eine Kamera (6) angeordnet ist, versehen zu werden,
- wenigstens eine mit den Antrieben gekoppelte Steuervorrichtung (35), welche eingerichtet ist, Signale zum Ansteuern der Antriebe zu erzeugen, damit die Befestigungsvorrichtungen (42, 43, 47) den Signalen zugeordnete Bewegungen durchführen, und
- eine mit der Steuervorrichtung (35) gekoppelte Bedienkonsole (36), die eine mit der Steuervorrichtung (35) gekoppelte manuelle Eingabevorrichtung (39, 40) und eine mit der Steuervorrichtung (35) gekoppelte Anzeigevorrichtung (38) aufweist,
**dadurch gekennzeichnet, dass** die Steuervorrichtung (35) eingerichtet ist, aufgrund eines Bildes vom Inneren des Lebewesens, das mit der während eines medizinischen Eingriffs in das Innere des Lebewesens (31) eingeführten Kamera (6) aufgenommen wurde, einen Volumendatensatz durch Fusionieren des dem Bild zugeordneten Bilddatensatzes mit einem vor dem Eingriff präoperativ aufgenommenen Volumendatensatzes vom Inneren des Lebewesens (31) zu erstellen, und den Volumendatensatz derart zu drehen, um das dem Volumendatensatz zugeordnete virtuelle Bild (21) vom Inneren des Lebewesens (31) aus einem vom Blickwinkel (8) der Kamera (6) unterschiedlichen virtuellen Blickwinkel (28) auf der Anzeigvorrichtung (38) anzuzeigen.

10. Medizinischer Arbeitsplatz nach Anspruch 9, dessen Steuervorrichtung (35) eingerichtet ist, den Volumendatensatz derart zu drehen, dass der virtuelle Blickwinkel (28) in Richtung des wenigstens einen medizinischen Instruments (2, 3) gerichtet ist.
